# EUROPEAN PATENT SPECIFICATION

(11) **EP 2 575 715 B1**
(45) Date of publication and mention of the grant of the patent: **29.10.2014**
(21) Application number: 11726543.9
(22) Date of filing: 25.05.2011
(51) Int. Cl.: A61F 9/007

(54) **DEVICE FOR PLACING CIRCUMFERENTIAL IMPLANT IN SCHLEMM'S CANAL**
VORRICHTUNG FÜR DEN EINSATZ EINES UMLAUFENDEN IMPLANTATS IN EINEN SCHLEMM-KANAL
DISPOSITIF DE POSITIONNEMENT D'UN IMPLANT CIRCONFÉRENTIEL DANS LE CANAL DE SCHLEMM

(30) Priority: 27.05.2010 US 348915 P
(43) Date of publication of application: 10.04.2013
(73) Proprietor: Ellex-iScience, Inc., Menlo Park CA 94025 (US)
(72) Inventor: YAMAMOTO, Ronald, K., San Francisco, California 94117 (US); CONSTON, Stanley, R., San Carlos, California 94070 (US)
(74) Representative: Neilson, Martin Mark
(86) International application number: PCT/US2011/037882
(87) International publication number: WO 2011/150045

(56) References cited:
- WO-A1-2008/002377
- WO-A1-2011/057830
- WO-A2-2006/066103
- US-A1- 2006 155 300
- US-B1- 6 524 275

## Description

### FIELD OF THE INVENTION

The present invention relates to devices for insertion of flexible elongated implants within the full circumference or a segment of the circumference of Schlemm's canal of an eye.

### BACKGROUND OF THE INVENTION

Glaucoma is a disease condition of the eye in which increased intraocular pressure (IOP) is created by blockage of the drainage mechanism for the aqueous fluid produced in the anterior portion of the eye. Such conditions are usually treated by topical drugs in the form of eye drops, but may result in surgical treatment if drug treatment becomes ineffective or if patient compliance is an issue. Traditional glaucoma surgery, known as a trabeculectomy, involves dissection of the eye and the forming of a fistula from the anterior chamber to the subconjunctival space. Trabeculectomy is associated with a high incidence of post-operative complications.

Recently developed surgical treatments for glaucoma have focused on restoration of the natural drainage system, including the trabecular meshwork and Schlemm's canal. The use of an implant that is placed in the entire circumference of Schlemm's canal to treat glaucoma is described in US Patent Application Publication No. 20060195187, published August 31, 2006 in the names of Stegmann et al. The device of the present invention provides novel surgical instruments that enable placement of an implant in the full circumference or segment of the circumference of Schlemm's canal, without penetration of the intraocular space.

### SUMMARY OF THE INVENTION

The present invention provides devices as defined in the claims that enable placement of an implant within the full circumference or a segment of the circumference of Schlemm's canal of an eye. An embodiment of a device according to the invention comprises a flexible elongated solid element with a proximal end and a distal tip that transmits light such as one or more strands of a fiber optic. The elongated solid element has suitable dimensions and appropriate mechanical characteristics to allow advancement within Schlemm's canal. The fiber optic element transmits light from a proximal connector to the distal tip of the elongated solid element to provide a lighted tip that may be viewed transclerally by the surgeon when the device is advanced along Schlemm's canal. This feature allows the surgeon to guide the device and avoid advancement into the wrong tissue spaces. The device is provided with fixation features at the distal tip that allow attachment of an implant to be pulled into Schlemm's canal by the device. Embodiments of such features may comprise an eyelet, a slot, a loop of material, or a circumferential groove at the distal tip or a bulbous tip of greater diameter than the elongated solid element of the device to which one end of a circumferential implant may be attached. By *solid* it is meant that there is a cross-section of solid material in the element such that there is no lumen extending on a longitudinal axis within the element.

In a cross-section through the eye, Schlemm's canal presents a flattened, narrow channel disposed at approximately 45° to the ocular axis with a major cross-sectional dimension of approximately 200 to 250 microns. The circumference of Schlemm's canal in a human eye is typically 36 mm. The elongated solid element of the device of the present invention is sized to fit within Schlemm's canal and has sufficient flexibility to adapt to the curvature of the canal during advancement. A rounded or atraumatic distal tip further aids the ability of the device to be advanced within the canal. The elongated solid element will have sufficient rigidity to be advanced along the lumen of Schlemm's canal by application of force at one or both ends of the elongated solid element without collapsing the elongated solid element within the canal. The elongated solid element will also have sufficient flexibility to bend to follow the tract of Schlemm's canal while the element is advanced into or retracted from Schlemm's canal without causing undue bleeding or tissue damage.

Typically a measurable flexural rigidity of the elongated solid element in the range of 2.2 x E-12 to 3.0 x E-10 kN*m² is useful. The elongated solid element may be made from metal, synthetic polymers such as plastics, natural fibers or polymers, or combinations thereof.

Optionally, the elongated solid element comprises a plurality of fiber optics.

A method using the devices of the invention for full circumferential insertion of an implant into Schlemm's canal of the eye comprises the steps of:
a) advancing fully circumferentially in Schlemm's canal a tool by movement the tool in a first direction through Schlemm's canal, the tool comprising a flexible elongated solid element with a distal and proximal ends, the distal and/or proximal end comprising a mechanical element for attachment of the implant, and a distal and/or proximal light-transmitting element for locating the distal and/or proximal end of the tool during placement and advancement, whereby the distal end is exposed upon exit from Schlemm's canal ;
b) attaching the proximal end of an implant at the exposed distal end of the tool, the implant comprising a second elongated element with distal and proximal ends and being a size sufficient for insertion within the canal and sufficient length for full 360° circumferential insertion into Schlemm's canal;
c) withdrawing the tool and attached implant through Schlemm's canal in the reverse direction of the first direction, whereby the tool is withdrawn from Schlemm's canal, the implant is fully circumferentially positioned within Schlemm's canal, and the attached proximal end of the implant is exposed upon exit from Schlemm's canal; and
d) detaching the implant from the tool.

After detaching the implant from the tool, the distal and proximal ends of the implant may be secured to each other directly or to a securement element, or to tissue.

### BRIEF DESCRIPTION OF THE DRAWINGS

Fig. 1 shows a device comprising a twisted optical fiber forming a terminal loop at the distal end.
Fig. 2 shows a device comprising an optical fiber with an atraumatic tip.
Fig. 3 shows a device comprising an optical fiber comprising a split end rejoined at an atraumatic tip.
Fig. 4 shows a device comprising a single optical fiber twisted back on itself at the distal end to form a loop.
Fig. 5 shows device comprising a single optical fiber with a rounded atraumatic tip incorporating a circumferential groove.
Fig. 6 shows a device after advancement into the full length of Schlemm's canal.

The invention is defined by the features of the claims.

### DESCRIPTION OF SPECIFIC EMBODIMENTS

To utilize the device a surgical access is created to expose Schlemm's canal by dissection of the overlying sclera. The device is placed within the surgical ostia of the canal and manually advanced. The light from the distal tip may be observed to insure the device is in the proper location of Schlemm's canal and continued to be advanced until the device distal tip passes through the entire canal and exits through the surgical access site. A lubricious friction reducing or hydrophilic coating on the device may be incorporated to reduce the force required to advance the device within Schlemm's canal.

The elongated solid element of the device has suitable dimensions and appropriate mechanical characteristics to allow advancement within Schlemm's canal. A solid elongated element is mechanically preferred as compared to a hollow elongated element which may kink or collapse due to axial or flexural loading forces during use. The elongated element preferably has length of at least 36 mm so that is may be inserted through the entire circumference of Schlemm's canal and expose the distal tip to the surgeon in order to perform attachment of an implant. The implant preferably has distal and proximal ends and a length and diameter sufficient for insertion within Schlemm's canal for full circumferencial insertion into the canal. The elongated solid element with implant attached may then be withdrawn back through Schlemm's canal until the attached end (proximal end) of the implant is exposed to the surgeon. The device may then be detached from the implant. The proximal and distal ends of the implant may be secured to each other directly or to a securement element, or to tissue, at the option of the surgeon. Similarly, the device may be used to position a length of suture within the circumference of Schlemm's canal. One end of the suture may be attached to the proximal end of the implant and the opposite end of the suture used to pull the implant into position within the canal.

The elongated solid element of the device will have typical dimensions of a diameter in the range of about 10-300 microns and a length of at least about 36 mm. Implants will typically have similar or smaller diameters than the elongated solid element of the device.

In one embodiment, the device comprises a length of flexible fiber optic folded over itself, then twisted to form an elongated solid element with a loop forming the distal tip. The loop provides a bend in the fiber optic where the critical incidence angle for total internal reflection is exceeded, and acts as a light source. Since there is no cut end of the fiber optic at the distal end, the loop also serves as an atraumatic tip and an eyelet for the attachment of an implant which may be pulled into Schlemm's canal.

The twisted configuration of the fiber optic fiber comprising the elongated solid element provides several ways to tailor the flexural properties of the device. The material composition and diameter of the fiber optic may also be selected to provide the desired amount of flexural rigidity of the elongated element. In addition, the twist pitch of the configuration may be adjusted to further tailor the flexural rigidity and the axial compressive stiffness. To allow placement in the full circumference of Schlemm's canal, it is preferred that the flexural rigidity is as low as feasible to maximize flexibility. An outer flexible tubular jacket or sleeve may be placed over the device up to the distal tip to further tailor the mechanical properties and protect the fiber optics.

In one embodiment, the implant to be placed within Schlemm's canal may have an end which comprises a filament or a connector which may be threaded through an eyelet at the distal end of the device. The eyelet may be formed by a twisted fiber loop, a single fiber optic with a hole formed in the distal end, a single fiber optic split and rejoined at the distal end or a single fiber optic with the distal end formed back into a loop. The implant may be secured to the eyelet after the device has passed through the circumference of the canal and then pulled into place within Schlemm's canal. Alternatively, the implant may be secured to the eyelet at the distal end prior to the device being placed into Schlemm's canal and pulled into place while the device is passing through the canal circumference. Similarly, the end of the implant may be tied to a device which has a slot, loop of material or bulbous tip located at the distal end to allow secure attachment of the implant. When a bulbous tip is utilized it is preferred that the diameter of the tip will be at least 50% greater than the cross-sectional thickness of the device to avoid unwanted detachment by slippage of a suture, string, filament, etc. attached to the device. While not intending to be limited to the following, the types of implants contemplated to be inserted into Schlemm's canal utilizing a device according to the invention include elastic or non-elastic filaments, sutures, wires, strings, cords, coils, stents and fibers.

In another embodiment, the attachment features of the device may be formed at the proximal end. After advancing the device through the full circumference of Schlemm's canal, one end of the implant may be attached to the proximal end and the device continued to be advanced or pulled into the canal to place the implant along the full circumference. Alternatively, the implant may be advanced or pulled into a desired segment of the canal and released. The release may be facilitated by attachment of the implant to the device with a length of suture or filament which may be cut or untied when the implant is properly positioned within Schlemm's canal.

In another embodiment, the device may comprise a fiber optic with a rounded atraumatic tip and features to secure one end of an implant to either the distal or proximal end of the device. The fiber optic may comprise a flexible polymer surrounded by a second polymer in a tubular configuration to enhance the optical or mechanical properties of the fiber optic.

The fiber optic is coupled to a proximal connector which provides connection to an illumination source to provide the light input to the distal tip of the device. The distal fiber optic of the device which is sized to fit within Schlemm's canal may be optically coupled to a larger diameter fiber optic through a connector element. Alternately, the distal fiber optic may be coupled directly to the proximal connector. In another embodiment, the fiber optic may be directly coupled to the illumination source without a connector.

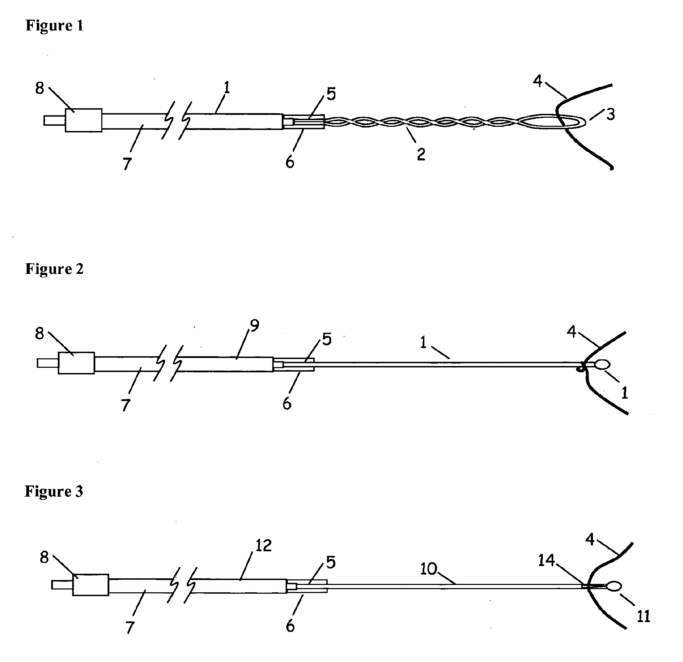

Figure 1 shows a detailed view of a device **1** comprising a flexible fiber optic **2** which has been twisted to form a loop **3** at the distal end for attachment of an implant device **4.** The proximal ends of the fiber optic **5** are placed into a connector **6.** The proximal ends of the fiber optic are optically coupled at the connector to the distal end of a second fiber optic **7** which terminates in a proximal connector **8** for attachment to an illumination source.

Figure 2 shows a detailed view of a device **9** comprising a single flexible fiber optic **10** wherein the distal tip is formed into a rounded atraumatic tip **11** with an implant device 4 attached. The proximal end of the fiber optic **5** is placed into a connector **6.** The proximal end of the fiber optic is optically coupled at the connector to the distal end of a second fiber optic **7** which terminates in a proximal connector **8** for attachment to an illumination source.

Figure 3 shows a detailed view of a device **12** comprising a single flexible fiber optic **10** wherein the distal segment has been split and rejoined **14** for attachment of an implant device **4.** The distal tip is formed into a rounded atraumatic tip **11.** The proximal end of the fiber optic **5** is placed into a connector **6.** The proximal end of the fiber optic is optically coupled at the connector to the distal end of a second fiber optic **7** which terminates in a proximal connector **8** for attachment to an illumination source.

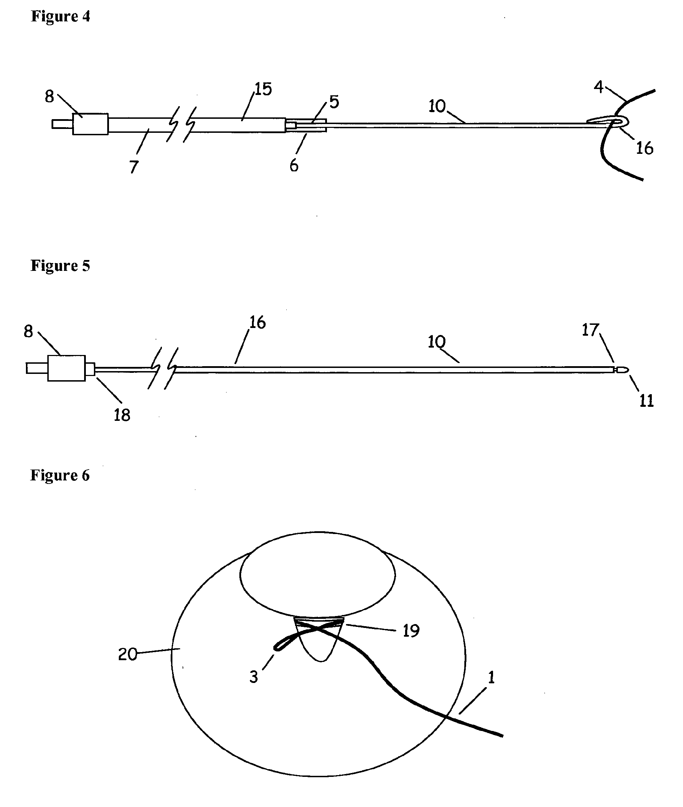

Figure 4 shows a detailed view of a device **15** comprising a single flexible fiber optic **10** wherein the distal end is formed back on itself in a loop **16** for attachment of an implant device **4.** The proximal end of the fiber optic **5** is placed into a connector **6.** The proximal end of the fiber optic is optically coupled at the connector to the distal end of a second fiber optic **7** which terminates in a proximal connector **8** for attachment to an illumination source.

Figure 5 shows a detailed view of a device **16** comprising a single flexible fiber optic **10** wherein the distal end is formed into a rounded atraumatic tip **11** which incorporates a circumferential groove **17** onto which an implant device (not shown) may be attached. The proximal end of the fiber optic **18** is coupled directly to a proximal connector **8** for attachment to an illumination source.

Figure 6 shows a schematic view of the device **1** of Figure 1 which has been advanced around Schlemm's canal **19** in an eye **20,** such that the distal loop **3** has exited the canal and is in position for attachment of an implant, so that the implant can be placed into the canal.

The following examples are presented for the purpose of illustration and are not intended to limit the invention in any way.

### EXAMPLES

### Example 1

Devices according to the invention were fabricated. Two device prototypes were constructed using 70 micron (0.0028 inch) and 100 micron (0.004 inch) outside diameter plastic optical fibers (Biogeneral Inc). The fibers comprised a polystyrene (PS) core, within a tubular layer of polymethylmethacrylate (PMMA) to act as cladding. The inner core and cladding were within a tubular jacket of polyvinylidene fluoride (PVDF). Fibers were cut to a length of 120 mm (4.7 inch) and the cut ends were aligned collinearly and joined together with UV curing adhesive (4305, Loctite Corp.) forming a tear-drop shaped loop. The joined ends were mounted into a rotary chuck and the looped end was placed over a 0.5 mm (0.02 inch) diameter shaft. As the rotary chuck was turned, UV curing adhesive with a durometer of 50 Shore D (201 CTH, Dymax Inc) was applied to the twisted fibers and cured in incremental lengths. The twisting was continued until the end loop was approximately 5 mm (0.2 inch) long.

The proximal ends were potted into a polycarbonate tube with UV adhesive (4305, Loctite Corp.). The resulting device was approximately 50 mm (2 inch) long. A larger plastic optical fiber (ESKA™ fiber, Mitsubishi Rayon Co LTD) was used to connect the prototype device to a laser diode fiberoptic illumination source (iLumin™, iScience Interventional Corp.) The fiber was comprised of a 250 micron (0.01 inch) diameter core of (poly) methyl methacrylate (PMMA), a fluorinated polymer cladding and a polyethylene jacket for a total outside diameter of 1 mm (0.04 inch). The jacket of the larger fiber was stripped to expose a short length of the core. The core was inserted into the polycarbonate connector until it butted against the cut ends of the twisted device fibers, and then adhesively bonded in place. The proximal end of the ESKA™ fiber terminated in a connector designed for the iLumin™ illuminator. The connector was plugged into the illuminator and the light source turned on. A bright light was seen at the distal loop end of the twisted fibers.

### Example 2

Another device according to the invention was fabricated. A plastic optical ESKA™ fiber with a 250 micron core as described in Example 1 was cut to a length of 500 mm (20 inch). The jacket was stripped from the core for a length of 50 mm (2 inch). The distal tip of the core was split with a razor blade. A 125 micron (0.005 inch) wire was inserted into the split to maintain the opening, while the distal cut ends were adhesively bonded back together with UV curing adhesive (4305, Loctite Corp). Additional adhesive was applied to the distal tip to create a ball end atraumatic tip of 340 microns (0.013 inch) diameter. The proximal end was joined to another length of the ESKA™ plastic fiber with a connector for the illuminator, as in Example 1. The device was plugged into the illuminator and a bright light was seen at the distal tip.

### Example 3

Additional devices according to the invention were fabricated. Devices comprising the 70 micron and 100 micron plastic optical fibers as described in Example 1 were used. UV cure adhesive (4305, Loctite Inc.) was used to form an olive shaped tip at the end of each fiber which was cut to a length of 50 mm (2 inch). The bulbous tips were nominally 325 microns (0.013 inch) diameter. The fibers were bonded end-to-end to a short length of bare ESKA™ fiber using the UV cure adhesive. The ESKA™ fiber was inserted into a polycarbonate connector attached to another length of jacketed ESKA™ fiber with a connector as in Example 1. When plugged into the illumination source (iLumin™, iScience Interventional Corp.), the devices exhibited a bright light at the distal tips.

### Example 4

The devices according to Example 1, Example 2 and Example 3 were tested in human cadaver eyes. Eyes were prepared using a standard two-flap scleral cut-down as used in non-penetrating glaucoma surgery to expose Schlemm's canal. The first device trial was performed using the 100 micron fiber loop from Example 1. The loop end was inserted into Schlemm's canal and advanced around the canal until the loop exited the surgical site. A 9-0 polypropylene suture (Prolene, Ethicon Inc) was inserted through the end loop and then the device was withdrawn through the canal, successfully pulling the suture into the canal. The second trial was performed with the 70 micron fiber loop from Example 1. The same method was used and the prototype successfully delivered the suture into the canal. It was noted that the smaller diameter fiber was somewhat more difficult to push around the canal but was still successful in advancement and then placement of the suture implant. The third trial used the device from Example 2. The larger and stiffer prototype was more difficult to advance around Schlemm's canal but was successful in transiting the full length. The fourth and fifth trials were performed with the prototype devices from Example 3. Each device was sufficiently flexible to be successfully advanced around Schlemm's canal until the distal tips emerged from the ostia of the canal. A 10-0 polypropylene suture (Prolene, Ethicon Inc) was tied to the distal ends of the devices and successfully withdrawn back through the canal. In each trial, the illuminated tip of the device was clearly seen through the scleral tissues and allowed for visual tracking of the device movement around Schlemm's canal.

### Example 5

Another device according to the invention was fabricated. The device comprised a 70 micron plastic optical fiber as described in Example 1. UV cure adhesive as described in Example 3 was used to form a bulbous tip of 175 microns in diameter. The fiber was bonded into a polycarbonate connector and ESKA™ fiber as in Example 1, and exhibited a bright light at the distal tip when plugged into the light source. A human cadaver eye was prepared and a surgical cut-down was made to expose Schlemm's canal. The tipped 70 micron fiber was inserted into the ostia of the canal and advanced 360° around the canal. The lighted tip was observed through the sclera as the fiber was advanced.

### Example 6

Plastic optical fibers of 70 and 100 micron diameters, described in Example 1, were evaluated using a mechanical tester (Instron) with a 5 Newton load cell to determine their flexural rigidity by 3-point bending. Flexural rigidity in bending was calculated from the output of the Instron. The tangent modulus in bending, E_{B}, was determined by using a modified ASTM D790-07 Flexural Test method. Due to the very small diameter of the fiber samples, the test method was modified by using smaller supports and a loading nose of 0.095 inch (2.4 mm) diameter and a smaller support span of 0.200 inch (5.08 mm). The Instron result of E_{B} was then multiplied by the 2nd moment of inertia, I, to yield the flexural rigidity, E*I. The moment, I, was calculated using I = π*r2 / 4, where r equals the radius of the fiber.

The small diameter optical fibers, when tested individually on the Instron, were below the detection limit for the load cell. To determine EB for the individual fibers, two fibers were adhesively bonded together in parallel with a low durometer UV cure adhesive (3321, Loctite Inc). The resulting tangent modulus was divided by two to yield E_{B}.

Twisted pairs of the 100 micron fiber, similar to Example 1, were tested to determine their flexural rigidity in the same manner. Two fibers were twisted together and then adhesively bonded as with the parallel fibers. Two different pitch twisted pairs were prepared, one with a 2 mm (0.08 inch) pitch and one with a 5 mm (0.2 inch) pitch. Table 1 shows the tested devices and their corresponding flexural rigidities. Devices with a flexural rigidity of 3.0 x 10⁻¹⁰ kNm² or less are sufficiently flexible to allow complete circumferential advancement of the device in Schlemm's canal.

**Table 1: Plastic Optical Fibers - Flexural Properties**

| **Test Sample** | **Flexural Rigidity (kN*m²)** |
|---|---|
| 70 um Fiber | 2.2 x 10⁻¹² |
| 100 um Fiber | 5.1 x 10⁻¹² |
| 100 um Twisted Pair, 2 mm Pitch | 1.5 X 10⁻¹⁰ |
| 100 um Twisted Pair, 5 mm Pitch | 3.0 x 10⁻¹⁰ |

### Example 7

An experiment was performed to evaluate the requirements for the diameter of the bulbous tip of a cannula in order to secure a small diameter suture which may act as an implant or may be attached to a separate implant device to effect placement of the implant within Schlemm's canal. Samples of model device tips were prepared by applying a small amount of adhesive (Loctite 4305, Loctite Corp) to the end of a 200 um (0.008 inch) 304 stainless steel wire. The adhesive was carefully applied to create smooth bulbous tips of varying diameter. A wire without a bulbous tip was also tested as a control. Segments of 10-0 Prolene suture (Ethicon, Inc) with a diameter of 30 um (0.0012 inch) were tied tightly to the wire samples and the suture loop was positioned just below the tip of the wire under test. An Instron mechanical tester with a 5 Newton load cell was used to measure the load required to pull the sutures from the wires, at a cross-head speed of 100 mm/min. Five runs were performed for each wire/tip sample and the results were averaged. The results are shown in the table 2 below. The "Break/Pull" column indicates how many sutures broke along the fiber without being pulled off the tip. The "% of Suture" column indicates the relative size of the bulbous tip radius in relation to the 30 micron suture, i.e. the 230 micron tip diameter represents a tip that extends radially from the wire with 1/2 of the suture diameter. For securement of the suture, the tip diameter should be sized to be larger than the cannula diameter where it interfaces the bulbous tip by more than 50% of the suture diameter.

**Table 2: Bulbous Tip Suture Securement Test Results**

| **Tip Dia (um)** | **Difference Between Tip and Cannula Diameters (um)** | **Break/Pull** | **% of Suture** | **Ave Load (gf)** | **Load Std Dev** |
|---|---|---|---|---|---|
| 200 | 0 | 0/5 | N/A | 1.5 | 0.3 |
| 230 | 30 | 0/5 | 50% | 11.5 | 2.3 |
| 245 | 45 | 2/5 | 75% | 24.5 | 6.5 |
| 270 | 70 | 3/5 | 117% | 25.7 | 2.1 |
| 300 | 100 | 5/5 | 167% | 41.5 | 4.7 |

## Claims

1. A tool for full circumferential insertion of an implant into Schlemm's canal (19) of the eye (20), said tool comprising:
a flexible elongated solid element with a distal end and proximal end, said distal and/or proximal end comprising a mechanical element for attachment of said implant;
**characterized by**:
a light-transmitting fiber optic (2) element folded over itself to form a loop (3) at the distal and/or proximal tip of said flexible elongated solid element whereby the loop (3) provides an atraumatic distal and/or proximal tip and provides a bend in the fiber optic (2) element such that the critical incidence angle for internal reflection is exceeded to produce a light source element for locating the distal and/or proximal end of said tool during placement and advancement.

2. The tool according to Claim 1 wherein said elongated solid element provides full 360° circumferential insertion of said element into Schlemm's canal (19).

3. The tool according to Claim 1 wherein said tool with an attached implant to said distal end provides full 360° circumferential insertion of said implant into Schlemm's canal (19).

4. The tool according to Claim 1 further comprising a lubricious coating.

5. The tool according to Claim 1 wherein said loop (3) forms an eyelet serving as said mechanical element for attaching said implant to said elongated solid element.

6. The tool according to Claim 1 wherein said mechanical element comprises a split and rejoined end of said fiber optic (2).

7. The tool according to Claim 1 wherein said mechanical element comprises a hole at an end of said fiber optic (2).

8. The tool according to Claim 1 wherein said elongated element is encased at least in part with a flexible tubular sleeve.

9. The tool according to Claim 1 wherein the elongated solid element has a flexural rigidity in the range of 2.2 x E-12 to 3.0 x E-10 kN*m².

10. The tool according to Claim 1 wherein said elongated solid element comprises a plurality of fiber optics (2).

11. The tool according to Claim 1 wherein said tool comprises a polymer.

12. A device for full circumferential insertion of an implant into Schlemm's canal (19) of the eye (20) comprising the tool according to claim 1
wherein an implant is attached to said tool, said implant comprising a second elongated element with distal and proximal ends and being a size sufficient for insertion within the canal (19) and sufficient length for full 360° circumferential insertion into Schlemm's canal (19).

13. The device according to Claim 12 wherein the elongated solid element has a flexural rigidity in the range of 2.2 x E-12 to 3.0 x E-10 kN*m².

14. The device according to Claim 12 wherein said implant comprises a filament.

15. The device according to Claim 14 wherein said filament comprises non-elastic material or elastic material.

## Patentansprüche

1. Werkzeug für eine volle umlaufende Einbringung eines Implantats in einen Schlemm-Kanal (19) des Auges (20), wobei das Werkzeug umfasst:
ein flexibles verlängertes festes Element mit einem entfernten und einem nahen Ende, wobei das entfernte und/oder das nahe Ende ein mechanisches Element zur Befestigung des Implantats umfasst;
**gekennzeichnet durch**:
ein lichtdurchlässiges Glasfaserelement (2), das über sich selbst gefaltet ist, um eine Schlaufe (3) an der entfernten und/oder nahen Spitze des flexiblen verlängerten festen Elements auszubilden, wobei die Schlaufe (3) eine atraumatische entfernte und/oder nahe Spitze bereitstellt und eine Biegung in dem Glasfaserelement (2) bereitstellt, sodass der kritische Einfallswinkel für eine interne Reflexion überschritten wird, um ein Lichtquellenelement zur Lokalisierung des entfernten und/oder nahen Endes des Werkzeugs während der Platzierung und des Vorrückens zu produzieren.

2. Werkzeug nach Anspruch 1, wobei das verlängerte feste Element eine volle 360° umlaufende Einbringung des Elements in den Schlemm-Kanal (19) bereitstellt.

3. Werkzeug nach Anspruch 1, wobei das Werkzeug mit einem Implantat, das an dem entfernten Ende befestigt ist, eine volle 360° umlaufende Einbringung des Implantats in den Schlemm-Kanal (19) bereitstellt.

4. Werkzeug nach Anspruch 1, weiterhin umfassend eine gleitfähige Beschichtung.

5. Werkzeug nach Anspruch 1, wobei die Schlaufe (3) eine Öse ausbildet, die als mechanisches Element zur Befestigung des Implantats mit dem verlängerten festen Element dient.

6. Werkzeug nach Anspruch 1, wobei das mechanische Element ein aufgeteiltes und wieder zusammengefügtes Ende der Glasfaser (2) umfasst.

7. Werkzeug nach Anspruch 1, wobei das mechanische Element ein Loch an einem Ende der Glasfaser (2) umfasst.

8. Werkzeug nach Anspruch 1, wobei das verlängerte Element mindestens teilweise mit einem flexiblen röhrenförmigen Ärmel eingeschlossen ist.

9. Werkzeug nach Anspruch 1, wobei das verlängerte feste Element eine Biegesteifigkeit im Bereich von 2,2 x 10⁻¹² bis 3,0 x 10⁻¹⁰ kN*m² hat.

10. Werkzeug nach Anspruch 1, wobei das verlängerte feste Element eine Vielzahl von Glasfasern (2) umfasst.

11. Werkzeug nach Anspruch 1, wobei das Werkzeug ein Polymer umfasst.

12. Vorrichtung zur vollen umlaufenden Einbringung eines Implantats in einen Schlemm-Kanal (19) des Auges (20), umfassend das Werkzeug nach Anspruch 1, wobei ein Implantat an dem Werkzeug befestigt ist, und wobei das Implantat ein zweites verlängertes Element mit entfernten und nahen Enden umfasst und eine Größe hat, die ausreichend ist zur Einbringung in den Kanal (19) und eine ausreichende Länge zur vollen 360° umlaufenden Einbringung in den Schlemm-Kanal (19) hat.

13. Vorrichtung nach Anspruch 12, wobei das verlängerte feste Element eine Biegesteifigkeit im Bereich von 2,2 x 10⁻¹² bis 3,0 x 10⁻¹⁰ kN*m² hat.

14. Vorrichtung nach Anspruch 12, wobei das Implantat ein Filament umfasst.

15. Vorrichtung nach Anspruch 14, wobei das Filament ein nicht-elastisches Material oder ein elastisches Material umfasst.

## Revendications

1. Outil pour l'insertion circonférentielle totale d'un implant dans le canal de Schlemm (19) de l'oeil (20), ledit outil comprenant :
un élément souple, solide et allongé avec une extrémité distale et une extrémité proximale, ladite extrémité distale et/ou ladite extrémité proximale comprenant un élément mécanique pour la fixation dudit implant ;
**caractérisé par** :
un élément fibre optique (2) transmettant une lumière, plié sur lui-même pour former une boucle (3) au niveau de la pointe distale et/ou de la pointe proximale dudit élément souple, solide et allongé, la boucle (3) fournissant ainsi une pointe distale et/ou une pointe proximale atraumatiques et fournissant une courbure dans l'élément fibre optique (2) de telle sorte que l'angle d'incidence critique pour la réflexion interne est dépassé pour fournir un élément source de lumière pour positionner l'extrémité distale et/ou l'extrémité proximale dudit outil durant le positionnement et la progression.

2. Outil selon la revendication 1, dans lequel ledit élément solide allongé permet l'insertion circonférentielle totale à 360° dudit élément dans le canal de Schlemm (19).

3. Outil selon la revendication 1, dans lequel ledit outil avec un implant fixé à ladite extrémité distale permet l'insertion circonférentielle totale à 360° dudit implant dans le canal de Schlemm (19).

4. Outil selon la revendication 1, comprenant en outre un revêtement lubrifiant.

5. Outil selon la revendication 1, dans lequel ladite boucle (3) forme un oeillet faisant office dudit élément mécanique pour la fixation dudit implant audit élément solide allongé.

6. Outil selon la revendication 1, dans lequel ledit élément mécanique comprend une extrémité fendue et réunie de ladite fibre optique (2).

7. Outil selon la revendication 1, dans lequel ledit élément mécanique comprend un trou au niveau d'une extrémité de ladite fibre optique (2).

8. Outil selon la revendication 1, dans lequel ledit élément allongé est enveloppé au moins partiellement d'un manchon tubulaire souple.

9. Outil selon la revendication 1, dans lequel l'élément solide allongé a une rigidité en flexion dans la plage de 2,2 x E-12 à 3,0 x E-10 kN*m².

10. Outil selon la revendication 1, dans lequel ledit élément solide allongé comprend une pluralité de fibres optiques (2).

11. Outil selon la revendication 1, dans lequel ledit outil comprend un polymère.

12. Dispositif pour l'insertion circonférentielle totale d'un implant dans le canal de Schlemm (19) de l'oeil (20) comprenant l'outil selon la revendication 1
dans lequel un implant est fixé audit outil, ledit implant comprenant un second élément allongé avec des extrémités distale et proximale et étant d'une taille suffisante pour l'insertion à l'intérieur du canal (19) et d'une longueur suffisante pour l'insertion circonférentielle totale à 360° dans le canal de Schlemm (19).

13. Dispositif selon la revendication 12, dans lequel l'élément solide allongé a une rigidité en flexion dans la plage de 2,2 x E-12 à 3,0 x E-10 kN*m².

14. Dispositif selon la revendication 12, dans lequel ledit implant comprend un filament.

15. Dispositif selon la revendication 14, dans lequel ledit filament comprend un matériau non élastique ou un matériau élastique.
